# EUROPEAN PATENT SPECIFICATION

(11) **EP 3 864 176 B1**
(45) Date of publication and mention of the grant of the patent: **22.01.2025**
(21) Application number: 19786967.0
(22) Date of filing: 11.10.2019
(51) Int. Cl.: C12Q 1/6883

(54) **METHOD FOR PREDICTION OF RESPONSE TO BONE MARROW STEM CELL THERAPY**
VERFAHREN ZUR VORHERSAGE DES ANSPRECHENS AUF EINE KNOCHENMARKSTAMMZELLTHERAPIE
PROCÉDÉ DE PRÉDICTION DE RÉPONSE À UNE THÉRAPIE DE CELLULES SOUCHES DE MOELLE OSSEUSE

(30) Priority: 12.10.2018 DE 102018125324
(43) Date of publication of application: 18.08.2021
(73) Proprietor: Universität Rostock, 18051 Rostock (DE)
(72) Inventor: STEINHOFF, Gustav, 18211 Rethwisch-Börgerende (DE); WOLFIEN, Markus, 18198 Kritzmow (DE)
(74) Representative: Beckord & Niedlich Patentanwälte PartG mbB
(86) International application number: PCT/EP2019/077650
(87) International publication number: WO 2020/074726

(56) References cited:
- WO-A1-2011/088125
- WO-A2-2009/061382
- DE-A1- 102017 107 661
- FLISTER MICHAEL J. ET AL: "SH2B3 Is a Genetic Determinant of Cardiac Inflammation and Fibrosis", CIRCULATION: CARDIOVASCULAR GENETICS, vol. 8, no. 2, 1 April 2015 (2015-04-01), US, pages 294 - 304, XP055920853, ISSN: 1942-325X, Retrieved from the Internet <URL:http://dx.doi.org/10.1161/CIRCGENETICS.114.000527> DOI: 10.1161/CIRCGENETICS.114.000527
- LAURA VELAZQUEZ: "The Lnk Adaptor Protein: A Key Regulator of Normal and Pathological Hematopoiesis", ARCHIVUM IMMUNOLOGIAE ET THERAPIAE EXPERIMENTALIS, BIRKHAEUSER-VERLAG, BASEL, CH, vol. 60, no. 6, 19 September 2012 (2012-09-19), pages 415 - 429, XP035133221, ISSN: 1661-4917, DOI: 10.1007/S00005-012-0194-X
- STEINHOFF GUSTAV ET AL: "Cardiac Function Improvement and Bone Marrow Response -: Outcome Analysis of the Randomized PERFECT Phase III Clinical Trial of Intramyocardial CD133<+>Application After Myocardial Infarction", EBIOMEDICINE,, vol. 22, 1 August 2017 (2017-08-01), pages 208 - 224, XP002781037, ISSN: 2352-3964

## Description

The present invention relates to a method for prediction of response to disease therapy, in particular to tissue regeneration, preferably to cardiovascular regeneration comprising the use of a biomarker. Further, the present invention relates to a combination of biomarkers for use in a method for prediction of response to disease therapy, in particular to tissue regeneration, preferably to cardiovascular regeneration, a computer device to perform a method according to the present invention and a device adapted for carrying out the inventive method.

Regenerative therapies for the repair of organic tissue, in particular of heart tissue, have been at the forefront of preclinical and clinical development in the last 17 years. In view of heart tissue, among the different approaches the direct application of bone marrow stem cells (BMSC) in heart tissue still has the most dedicated clinical developmental attention since the first-in-man application in 2001 and the initial promising clinical trials (Stamm C, Westphal B, Kleine HD, et al. Lancet. 2003; 361(9351):45-46; Tse HF, Kwong YL, Chan JK, Lo G, Ho CL, Lau CP. Lancet 2003; 361(9351):47-9; Stamm C, Kleine HD, Choi YH, et al. J Thorac Cardiovasc Surg 2007;133(3):717-25). Yet, in these trials, clinically relevant improvements of LVEF (Left Ventricular Ejection Fraction) as well as non-responsive patients could be observed both in treatment and placebo groups (Timothy D H, Lem M, Jay H T, Circulation Research 2016; 119:404-406; Nasseri B A, Ebell W, Dandel M, et al. Eur Heart J. 2014, 35(19):1263-74; Bartunek J, Terzic A, Davison B A et al. Eur. Heart J. 2016 Dec 23. pii: ehw543. doi: 10.1093/eurheartj/ehw543).

Hematopoietic stem cells (HCSs) are the stem cells that give rise to other blood cells. In vertebrates, the vast majority of hematopoiesis occurs in the bone marrow (BM) and is derived from a limited number of hematopoietic stem cells that are multipotent and capable of extensive self-renewal. The hematopoietic system has traditionally been considered unique among phenotypically characterized adult stem/progenitor cells (Slack JM. Science 2000; 287:1431-1433; Blau HM, Brazelton TR, Weimann JM. Cell 2001;105:829-841; Korbling M, Estrov Z. New England Journal of Medicine 2003; 349:570-582) in that it is an organized, hierarchic system with multipotent, self-renewing stem cells at the top, lineage-committed progenitor cells in the middle, and lineage-restricted precursor cells, which give rise to terminally differentiated cells, at the bottom (Weissmann IL. Cell 2000; 100:157-168). However, disorders of clonal hematopoiesis have been described in hematological and cardiovascular disease patients and associated to congenital or somatic DNA mutations (Moehrle BM, Geiger H. Exp Hematol 2016; Oct; 44(10):895-901. doi: 10.2016/j.exphem.2016.06.253. Epub 2016 Jul 8; Jaiswal S, Natarajan P, Silver AJ et al. N Engl J Med 2017; Jul 13; 377(2):111-121. doi: 10.1056/NEJMoa1701719. Epub 2017 Jun 21.). The question arises, which mutations in stem congenital or somatic cell regulatory genes cause hematopoietic clonal advantage and impact cardiovascular pathology (Machiela MJ, Chanock SJ. Curr Opin Genet Dev 2017; Feb;42:8-13. doi: 10.1016/j.gde.2016.12.002. Epub 2017 Jan 6.).

One of the major genes associated with hematopoetic stem cell (HSC) proliferation disorders, such as myelodysplasia, erythrocytosis or leukemia by somatic mutation, is SH2B3 coding for the lymphocyte adapter protein (Lnk, LNK) adaptor protein (Elias HK, Bryder D, Park CY. Semin Hematol 2017; Jan; 54(1):4-11. doi: 10.1053/j.seminhematol.2016.11.002. Epub 2016 Nov 20.). However, Lnk/SH2B3 expression regulation in human bone marrow (BM) and clonal blood stem and progenitor cells is largely unknown for cardiovascular regeneration (Steinhoff G, Nesteruk J, Wolfien M, Große J, Ruch U, Vasudevan P, Müller P. Adv Drug Deliv Rev 2017; Oct 1; 120:2-24. doi: 10.1016/j.addr.2017.10.007. Epub 2017 Oct 18; Zhu X, Fang J, Jiang DS, Zhang P, Zhao GN, Zhu X, Yang L, Wei X. 2-B3Hypertension 2015; Sep; 66(3):571-81. doi: 10.1161/HYPERTENSIONAHA.115.05183. Epub 2015 Jun 22.).

Lnk/SH2B3 shares a pleckstrin homology domain, a Src homology 2 domain, and potential tyrosine phosphorylation sites with APS and SH-2B. It belongs to a family of adaptor proteins implicated in integration and regulation of multiple signaling events (Ahmed Z, Pillay TS. Biochem J 2003; 371 :405-412., Huang X, Li Y, Tanaka K, Moore KG, Hayashi JI. Proc Natl Acad Sci USA 1995; 92:11618-11622; Takaki S, Watts JD, Forbush KA, Nguyen NT, Hayashi J, Alberola.lla J, Aebersold R, Perlmutter RM. Biol Chem 1997; 272:14562-14570.) and has also been suggested to act as a negative regulator in the stem cell factor (SCF)/-c-Kit signaling pathway (Moehrle BM, Geiger H. Exp Hematol 2016; Oct;44(10):895-901. doi: 10.1016/j.exphem.2016.06.253. Epub 2016 Jul. 8., Takaki S, Sauer K, Iritani BM, Chien S, Ebihara Y, Tsuji K, Takatsu K, Perlmutter RM. Immunity 2000; 13:599-609.). Lnk/SH2B3 has also been reported to play a critical role in maintaining the ability of HSCs to self-renew, in a study that based on BM c-Kit⁺/Sca-1⁺/Lineage (Lin)⁻ (KSL) CD34⁻ cells, which are a more immature HSC subpopulation than KSL CD34⁺ cells (Ema H, Sudo K, Seita J, Matsubara A, Morita Y, Osawa M, Takatsu K, Takaki S, Nakauchi H. Dev Cell 2005; 8:907-914.).

Takaki *et al.* reported that Lnk/SH2B3 is expressed in hematopoietic cell lineages in mice, and BM cells of Lnk/SH2B3-deficient mice are competitively superior in hematopoietic population to those of WT mice (Takaki S, Morita H, Tezuka Y, Takatsu K. Lnk/SH2B3. J Exp Med 2002; 195:151-160.). They also clarified that not only HSC/hematopoietic progenitor cell (HPC) numbers but also the self-renewal capacity of some HSCs/HPCs were markedly increased in Lnk/SH2B3^{-/-} mice (Ema H, Sudo K, Seita J, Matsubara A, Morita Y, Osawa M, Takatsu K, Takaki S, Nakauchi H. Dev Cell 2005; 8:907-914). In addition, they identified the functional domains of Lnk/SH2B3 and developed a dominant-negative Lnk/SH2B3 mutant that inhibits the functions of Lnk/SH2B3 endogenously expressed in the HSCs/HPCs and thereby potentiates the HPCs for engraftment (Takizawa H, Kubo-Akashi C, Nobuhisa I, Kwon SM, Iseki M, Taga T, Takatsu K, Takaki S.. Blood 2006; 107:2968-2975.).

DE 10 2017 107661 A1 describes SH2B3 for use as a diagnostic marker.

WO 2011/088125 A1 discloses methods, compositions and kits for screening a subject for a hematolymphoid neoplasm or malignancy, or as having a predisposition or higher susceptibility of having a hematolymphoid neoplasm or malignancy, by determining the sequence of the LNK gene, where the presence of a sequence variation as compared to the wild type sequence indicates that the subject has a hematolymphoid neoplasm or malignancy or a predisposition or higher susceptibility of developing a hematolymphoid neoplasm or malignancy.

Velazquez, L. (Arch. Immunol. Ther. Exp. (2012) 60: 415-429) report on the development and function of blood cells which are regulated by specific growth factors/cytokines, in particular the role of adaptor proteins in cytokine signaling regulation and their receptors' signaling pathways.

Flister Michael J. et al. (Circulation: Cardiovascular Genetics, vol. 8 (no. 2), 1 April 2015 (2015-04-01), pp. 294-304, XP055920853, ISSN: 1942-325X, DOI: 10.1161/CIRCGENETICS.114.000527) describe an experimental model of MI (left anterior descending artery occlusion/reperfusion injury) in wild-type and SH32B3 knockout rats (SH2B3(em2Mcwi)) to assess the role of Sh2b3 in post-MI fibrosis, leukocyte infiltration, angiogenesis, left ventricle contractility, and inflammatory gene expression.

The inventors have identified a method for prediction of response to disease therapy, in particular to tissue regeneration, preferably to cardiovascular regeneration, comprising the determination of a biomarker. The present invention is thus directed to a method for prediction of response to cardiovascular regeneration comprising the determination of a biomarker, according to claim 1 of the present application. Further, the present invention relates to a combination of biomarkers for use in a method for prediction of response to the disease therapy, in particular to tissue regeneration, preferably to cardiovascular regeneration, a computer device to perform such a method and a device adapted for carrying out the inventive method.

The present invention is derived from a method for prediction of response to disease therapy, in particular to cardiovascular regeneration, wherein the method comprises
(i) determining in a sample of a subject a gene and/or -gene expression as a biomarker, wherein the gene and/or -gene expression comprises a mutational variant,
(ii) comparing the determined gene and/or -gene expression mutational variant to a baseline value and/or a reference, preferable to a baseline value and/or a reference of non-disease type or individual reference cell without somatic mutation gene and/or -gene expression,
(iii) predicting, based on the results of the comparison whether a response to disease therapy, in particular to tissue repair, preferably to cardiac repair, in the subject is to be expected, not expected or is ambivalent.

The invention is directed to a method for prediction of response to cardiovascular regeneration, wherein the method comprises
(i) determining in a sample of a subject an amount of SH2B3-gene and/or -gene expression product as a biomarker, wherein the SH2B3-gene and/or -gene expression product comprises a mutational variant,
   wherein the SH2B3-gene and/or -gene expression variant comprises at least one mutation of SH2B3-gene and/or -gene expression Variant 1 and/or SH2B3-gene and/or -gene expression Variant 2,
   wherein the SH2B3-gene and/or -gene expression variant comprises a sequence according to SEQ ID NO: 1 and/or SEQ ID NO: 2,
(ii) comparing the determined amount of SH2B3-gene and/or -gene expression mutational variant product to a baseline value and/or a reference, preferable to a baseline value and/or a reference of SH2B3 non-disease type or individual reference cell without somatic mutation gene and/or -gene expression,
(iii) predicting, based on the results of the comparison whether a response to disease therapy, in particular to tissue repair, preferably to cardiac repair, in the subject is to be expected, not expected or is ambivalent.

Preferably, the method of the present invention is an ex *vivo* / *in vitro* method. Moreover, it may comprise steps in addition to those explicitly mentioned above. For example, further steps may relate to sample pre-treatments or further evaluations or uses of the results obtained by the method. The method may be carried out manually or assisted by automation. Preferably, step (i) may in total or in part be assisted by automation, e.g. by a suitable robotic and sensory equipment. Steps (ii) and/or (iii) may be assisted by data processing units which carry out the respective comparisons and/or predictions.

The SH2B3 gene encodes a member of the SH2B adaptor family of proteins, which are involved in a range of signaling activities by growth factor and cytokine receptors. The encoded protein is a key negative regulator of cytokine signaling and plays a critical role in hematopoiesis. Mutations in this gene have been associated with susceptibility to celiac disease type 13 and susceptibility to insulin-dependent diabetes mellitus. Alternatively spliced transcript variants encoding different isoforms have been found for this gene. SH2B3 is also identified under HGNC: 29605, Entrez Gene: 10019, Ensembl: ENSG00000111252, OMIM: 605093, UniProtKB: Q9UQQ2.

The term "predicting" as used herein means assessing the probability according to which a subject will benefit from disease therapy, in particular to tissue repair, preferably to cardiac repair, more preferably from cardiac stem cell therapy in that there is a functional improvement of the cardiovascular system as defined elsewhere herein in detail after the said cardiac therapy. Within the context of the present invention, the term cardiac repair and cardiac regeneration is used synonymously.

As will be understood by those skilled in the art, such an assessment is usually not intended to be correct for 100% of the subjects to be diagnosed. The term, however, requires that the assessment is correct for a statistically significant portion of the subjects (e.g. a cohort in a cohort study). Whether a portion is statistically significant can be determined without further ado by the person skilled in the art using various well known statistic evaluation tools, e.g., by determination of confidence intervals, p-value determination, Student's t-test, Mann-Whitney test etc. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

The term "cardiovascular regeneration" as used herein includes the regeneration and/or treatment and/or improvement of diseases related to the cardiovascular system.

The term "biomarker" refers to molecules, the presence, absence or amount of which correlates with medical conditions or predispositions. Biomarkers may be any molecules and/or cells or subpopulations thereof, which occur in the subject. Typically, biomarkers are proteins, peptides, small molecules or nucleic acids, such as DNAs or RNAs, or cells and subpopulations thereof. In accordance with the present invention, the term preferably, refers to a gene and/or -gene expression, wherein the gene and/or -gene expression comprises a mutational variant. Such a gene and/or -gene expression may be analysed by sequencing of DNA and/or RNA. Analysing the gene and/or -gene expression might also include coexpression of a further gene and/or -gene expression. Such a determination of gene and/or -gene expression might be performed, e. g. in cardiomyocytes.

Determining the amount of a biomarker referred to in this specification relates to measuring the amount or concentration, preferably, semi-quantitatively or quantitatively. Measuring can be done directly or indirectly. Direct measuring relates to measuring the amount or concentration of the biomarker based on a signal which is obtained from the biomarker itself and the intensity of which directly correlates with the number of molecules of the biomarker present in the sample. Such a signal - sometimes referred to herein as intensity signal - may be obtained, e.g. by measuring an intensity value of a specific physical or chemical property of the biomarker. Indirect measuring includes measuring of a signal obtained from a secondary component (i.e. a component not being the biomarker itself) or a biological read out system, e.g. measurable cellular responses, ligands, labels, or enzymatic reaction products.

In accordance with the present invention, determining the amount of the biomarker can be achieved by all known means for determining the amount of a peptide, a protein, a small molecule, nucleic acids such as DNAs or RNAs or a cell or subpopulations thereof, in a sample. Said means comprise immunoassays and methods, which may utilize labelled molecules in various sandwich, competition, or other assay formats. Such assays are, preferably, based on detection agents such as antibodies, which specifically recognize the biomarker to be determined. The detection agents shall be either directly or indirectly capable of generating a signal indicating the presence or absence of the biomarker. Moreover, the signal strength can, preferably, be correlated directly or indirectly (e.g. reverse-proportional) to the amount of biomarker present in a sample. Further suitable methods comprise measuring a physical or chemical property specific for the biomarker such as its precise molecular mass or NMR spectrum. Said methods comprise, preferably, biosensors, optical devices coupled to immunoassays, FACS analysis, biochips, analytical devices such as mass-spectrometers, NMR-analysers, or chromatography devices. Further, methods include micro-plate ELISA-based methods, fully-automated or robotic immunoassays, enzymatic Cobalt binding assays, and latex agglutination assays.

Preferably, determining the amount of the biomarker comprises the steps of (a) contacting a cell capable of eliciting a cellular response the intensity of which is indicative of the amount of the biomarker with the said biomarker for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the biomarker.

Also preferably, determining the amount of a biomarker comprises the step of measuring a specific intensity signal obtainable from the biomarker in the sample. As described above, such a signal may be the signal intensity observed at an m/z variable specific for the biomarker observed in mass spectra or an NMR spectrum specific for the biomarker.

The term "amount" as used herein encompasses the absolute amount of a biomarker, the relative amount or concentration of said biomarker as well as any value or parameter, which correlates thereto or can be derived therefrom. Such values or parameters comprise intensity signal values from all specific physical or chemical properties obtained from said peptides by direct measurements, e.g. intensity values in mass spectra or NMR spectra. Moreover, encompassed are all values or parameters which are obtained by indirect measurements specified elsewhere in this description, e.g. response levels determined from biological read out systems in response to the peptides or intensity signals obtained from specifically bound ligands. It is to be understood that values correlating to the aforementioned amounts or parameters can also be obtained by all standard mathematical operations and can be used without dimensions, e.g. in scoring systems as described elsewhere herein.

The term "comparing" as used herein encompasses comparing the amount of the biomarker comprised by the sample to be analysed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. However, in accordance with the present invention it is also envisaged to calculate a value based on the measured amounts of the biomarkers. Said value is compared to reference intervals which have been derived from a multivariate discriminant analysis performed on amounts from a collective of subjects comprising pre-defined responders and non-responders to cardiac stem cell therapy. Further details may be found in the accompanying examples, below.

The comparison referred to in the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format. Preferably, such an assessment is performed by machine learning (ML). Based on the comparison of the determined amounts and the reference, it is possible to predict a response to cardiac regeneration, e.g. a functional improvement of the heart after cardiac stem cell therapy. In particular, it shall be possible to predict whether there is a high probability (i.e. the subject will be a responder), a low probability (i.e. the subject will be a non-responder) or the subject is ambivalent. Therefore, the reference amount is to be chosen so that either a difference or a similarity in the compared amounts allows identifying those test subjects, which belong into the group of subjects exhibiting a symptom of acute inflammation having either an ischemic or non-ischemic cerebral damage. The method allows either excluding (rule-out) or identifying (rule-in) a subject as a subject suffering from (i) an ischemic cerebral damage or (ii) a non-ischemic cerebral damage.

The term "reference" as used herein refers to a value, threshold or interval based on amounts of the biomarker which allows for allocation of a subject into either the group of subjects which can be expected to benefit from the disease therapy, in particular from tissue repair, preferably from cardiac therapy, which can be expected not to benefit from the disease therapy, in particular from tissue repair, preferably from cardiac therapy, or those which are ambivalent.

Preferably, such a reference is a non-disease type or individual reference cell without somatic mutation gene and/or -gene expression. Even more preferably, such a reference is an SH2B3 non-disease type or individual reference cell without somatic mutation gene and/or -gene expression,

Such a reference can be a threshold amount, which separates these groups from each other. A suitable threshold amount separating the two groups can be calculated without further ado by the statistical tests referred to herein elsewhere based on amounts of the biomarkers from either a subject or group of subjects known to benefit from disease therapy, in particular to tissue repair, preferably to cardiac repair, or a subject or group of subjects known not to benefit from said therapy or those which are known to be ambivalent.

A reference can, in principle, also be calculated for a cohort of subjects as specified above based on the average or mean values for a given biomarker by applying standard statistically methods. In particular, accuracy and sensitivity of a test such as a method aiming to diagnose an event, or not, is best evaluated by its receiver-operating characteristics (ROC) (see especially Zweig 1993, Clin. Chem. 39:561-577). Accordingly, the reference to be used for the aforementioned method of the present invention may be generated by establishing a ROC for said cohort as described above and deriving a threshold amount therefrom. Dependent on a desired sensitivity and specificity for a diagnostic method, the ROC plot allows deriving suitable thresholds.

The term "sample" refers to a sample of a body fluid, and preferably, to a sample of (whole) blood, plasma or serum. The term, however, also encompasses all samples which are derived from the aforementioned whole blood, plasma or serum by, e.g. pre-treatment steps such as fractions of blood, plasma or serum obtained by, e.g. partial purification.

The term "subject" as used herein refers to an animal, preferably, a mammal and, most preferably, a human. The subject shall be in need of disease therapy, preferably of cardiac therapy. Preferably, a subject in need for cardiac therapy suffers from a cardiac disease such as heart failure or coronary artery disease, e.g. after myocardial infarction, and, more preferably, from heart failure.

The prediction made according to the method of the invention also allows for assessing whether the probability is high and, thus, it is expected that a functional improvement of the cardiac system in a subject occurs, or whether the probability is such that the therapy success is ambivalent or whether the probability is low and, thus, it is expected that a functional improvement of the cardiac system in a subject will not occur.

Also, the present invention is directed to a computer device comprising a processor and a memory encoding one or more machine learning (ML) models coupled to the processor, wherein said programs cause the processor to perform a method, said method comprising
(i) comparing the determined biomarker/s to a baseline value and/or a reference, preferably to a baseline value and/or a reference of SH2B3-gene and/or -gene expression without mutational variant,
(ii) predicting, based on the results of the comparison, whether a response to disease therapy, in particular to tissue regeneration, preferably to cardiovascular regeneration, in the subject is to be expected, not expected or is ambivalent.

In addition, the present invention pertains to a device adapted for carrying out the inventive method, comprising
(i) an analysing unit for determining in a sample of the subject the amount of each of the biomarker/s according to the present invention, and
(ii) a computer device according to the present invention (as detailed above).

The term "device" as used herein relates to a system comprising the aforementioned units operatively linked to each other as to allow the diagnosis according to the methods of the invention. Preferred detection agents, which can be used for the analysing unit, are disclosed elsewhere herein. Preferred detection agents are antibodies or other proteins that specifically bind to the biomarker(s) and form detectable complexes. The analysing unit, preferably, comprises said detection agents in immobilized form on a solid support which is to be contacted to the sample comprising the biomarkers the amount of which is to be determined. Moreover, the analysing unit can also comprise a detector, which determines the amount of detection agent, which is specifically bound to the biomarker(s). The determined amount can be transmitted to the evaluation unit. Said evaluation unit comprises a data processing element, such as a computer, with an implemented algorithm for carrying out a comparison between the determined amount and a suitable reference. Suitable references can be derived from samples of subjects to be used for the generation of reference amounts as described elsewhere herein above. The results may be given as output of parametric diagnostic raw data, preferably, as absolute or relative amounts. It is to be understood that these data will need interpretation by the clinician. However, also envisaged are expert system devices wherein the output comprises processed diagnostic raw data the interpretation of which does not require a specialized clinician.

Further preferred embodiments of the invention are derived from the dependent claims together with the following description, whereby the patent claims of a certain category may be formed by dependent claims of a different category, and features of the different examples may be combined to new examples. It is to be understood that the definitions and explanations of the terms made above and below apply accordingly for all embodiments described in this specification and the accompanying claims. In the following, particular embodiments of the method of the present invention are specified further.

According to the invention, the SH2B3-gene and/or -gene expression is a knock-out-gene and/or -gene expression variant. Preferably, such a knock-out-gene and/or -gene expression variant is a dominant negative SH2B3⁻/LNK⁻ variant or LNK/SH2B3 deficient to inhibit the function of endogeneously expressed LNK/SH2B3.

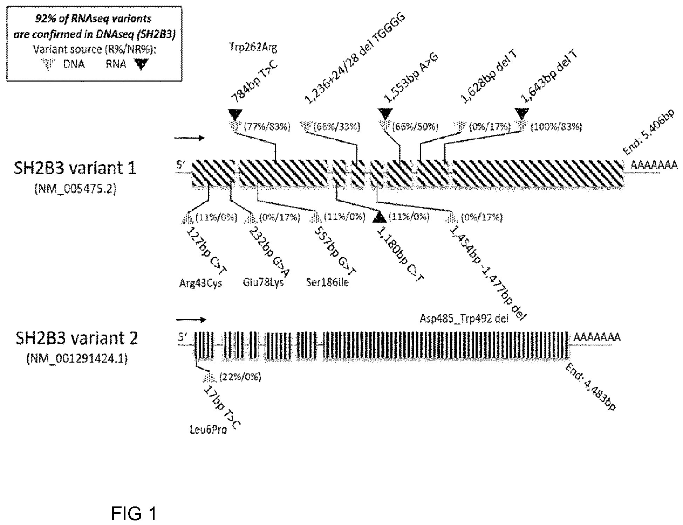

Specifically, such a variant is an SH2B3-gene and/or -gene expression variant that comprises at least one mutation of SH2B3-gene and/or -gene expression Variant 1 (Transcript: NM_005475.2) and/or SH2B3-gene and/or -gene expression Variant 2 (Transcript: NM_001291424.1), as depicted in Figure 1. Such an SH2B3-gene and/or -gene expression variant may be caused by SNP deletion and/or nucleotide exchange on the level of RNA and/or DNA and may, e. g. result in LNK protein to exhibit amino acid exchange in the range of 70 to 90 %, preferably in the range of 80 %, in particular about 83 %.

According to the invention, the SH2B3-gene and/or -gene expression Variant 1 comprises a sequence selected from SEQ ID NO:1; the SH2B3-gene and/or -gene expression Variant 2 comprises a sequence selected from SEQ ID NO:2. While SEQ ID NO:2 lacks two alternate exons in the 5' region, including a portion of the 5' coding region, and initiates translation at an alternate start codon in an alternate exon, compared to SEQ ID NO:1. The encoded isoform 2 has a distinct N-terminus and is shorter than isoform 1.

By using the above SH2B3-gene or -gene expression mutational variant together with a further biomarker, in particular a biomarker selected from CD133 gene expression and/or CD133+/ml MNC cells in peripheral blood and/or CFU-Hill, the method according to the present invention is beneficially capable to increase sensitivity and specificity of prediction accuracy to more than about 60 %.

A further preferred method uses analysis of LNK protein expression and function of an SH2B3-gene and/or -gene expression variant, thereby analysing inhibition and/or change of LNK function due to amino acid exchange or -deletion.

According to a specifically preferred embodiment, the method further uses one or more biomarker/s, wherein the further biomarkers is selected from the group of Angiogenic factor and/or Survival factor and/or Chemokine and/or circulation endothelial progenitor cells (EPC) and/or circulation endothelial cells (CEC) and/or circulating thrombocytes and/or circulating mononuclear cells and subpopulations, receptor/ligand expression on MNC subpopulations and/or whole genome sequence RNA.

According to a particularly preferred embodiment, the advantageous method further uses one or more biomarkers, wherein the further biomarker/s is/are selected from PLCG1, EPO, LPCAT2, GRB2, AP1B1, AFAP1, KLF8, MARK3, REX1BD, SACM1L, PDGFRB, VEGF, BEX3, Delta_CT_SH2B3, ZNF205, LTB, EMG1, CD34+ cells/ml PB, BAZ1A and/or CD133+ cells/ml PB.

Preferably, PLCG1, LPCAT2, GRB2, AP1B1, AFAP1, KLF8, MARK3, REX1BD, SACM1L, PDGFRB, BEX3, ZNF205, EMG1, BAZ1A and/or LTB is/are determined on an RNA level and/or EPO and/or VEGF is/are determined on a protein level.

Even more preferably, determination of EPO, VEGF, Delta_CT_SH2B3, CD34+ cells/ml PB and/or CD133+ cells/ml PB is conducted from preoperative samples.

According to an even more preferred embodiment the further biomarker/s is/are selected from PLCG1, EPO, LPCAT2, GRB2, AP1B1, AFAP1, KLF8, MARK3, REX1BD, SACM1L, PDGFRB and/or VEGF.

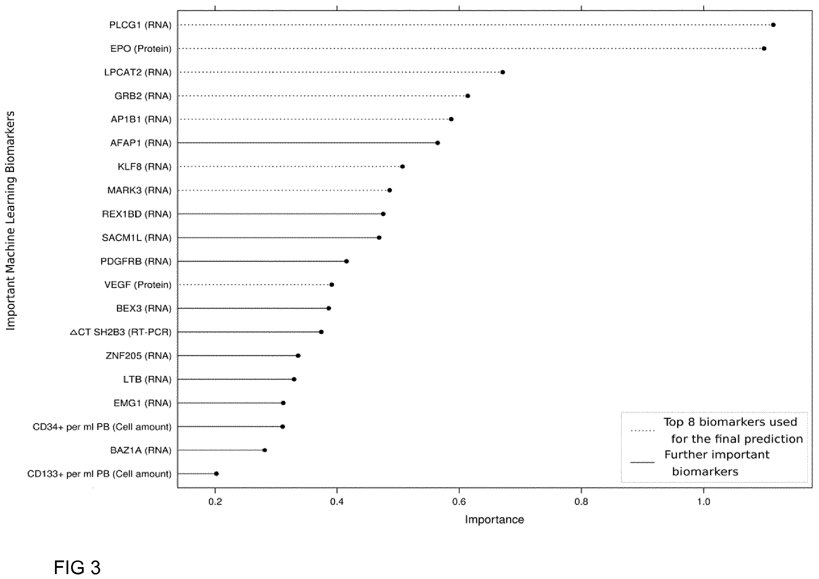

A combination of the above mentioned further biomarkers may be selected on an individual basis, depending on the disease type and/or disease state of the subject or patient, respectively. For example, a selection may encompass a combination of biomarkers including PLCG1, EPO, LPCAT2, GRB2, AP1B1, KLF8, MARK3 and/or VEGF (as depicted in Fig. 3).

In addition to the above mentioned further biomarker/s, according to a next embodiment, for example, the Angiogenic factor is selected from VEGF, preferably from VEGF-B, and/or FGF, preferably from FGF-4, and/or HGF and/or Ang-1, the Survival factor is preferably selected from IGF-1 and the Chemokine is selected from IGF-2 and/or SDF-1.

It has been advantageously found in the studies underlying the present invention that analysing the amount of a combination of the biomarkers in a sample taken prior to cardiac therapy from a subject in need of said cardiac therapy, e.g. a subject suffering from heart failure, allows for predicting whether that subject will benefit from the therapy in that the cardiac regeneration or cardiovascular repair is improved after therapy. The techniques for measuring the amounts of the individual biomarkers are all well known in the art.

Beneficially, by implementing the method according to the present invention, sensitivity and specificity of prediction accuracy can be higher than about 90 %, preferably higher than about 92 %, more preferably higher than about 93 %, most preferably higher than about 94 %. Thus, the method of the present invention improves decision making of clinicians prior to the application of an expensive and cumbersome therapeutic measure such as cardiac therapy. Making the right decision, i.e. applying the therapy only if it is effective, will certainly also be beneficial for the individual patients and, in light of the cost consequences, for the public health system as such.

By using ML, the method is preferably further assisted since no individual comparisons must be carried out. Furthermore, the machine learning approach beneficially serves to balance and weighting the parameters such that reliability of the prediction can be further improved. Thus, an advantageous method of the present invention is preferably assisted by machine learning to facilitate and improve the overall prediction accuracy. In accordance with the present invention, the term "machine learning" relates to the study and construction of algorithms that can learn from and make predictions on data - the algorithms hereby overcome strictly static program instructions by making data-driven predictions or decisions through building a model from sample inputs.

In another embodiment of the method, such method further uses one or more biomarker/s, wherein the one or more biomarkers are selected from zinc finger protein ZDHHC2 and/or MYL4/ALC1 and/or INPP4B and/or RBM38 and/or CD133+cells/ml PB (peripheral blood) and/or CD34+ cells/ml PB and/or CD45+184+ cells/ml PB and/or CD45+ cell/ml PB and/or CD184+ cells/ml PB and/or MNC in PB and/or CFU-Hill of PB and/or RIOK3-gene expression in PB and/or ABCC13-gene expression in PB and/or COPS3-gene expression in PB and/or GUK1-gene expression in PB and/or AP3B1-gene expression in PB and/or TBC1D22B-gene expression in PB and/or RBM38-gene expression in PB and/or ZBTB33-gene expression in PB and/or AGO2-gene expression in PB.

In another preferred embodiment, the method is used for preoperative prediction of response to stem cell therapy and/or induction of angiogenesis response and/or tissue repair in the context of cardiovascular disease including myocardial infarction, stroke and peripheral ischemic vascular disease, heart disease and/or ischemic preconditioning. Such a therapy may include a treatment to use a cardiovascular implant or stent, a ventricular assist device (VAD), a pacemaker and/or an occluder or appropriate closure device. Also, such a therapy may include treatment of diabetes mellitus, tumor diseases, coeliakie, rheumatic diseases, infectious diseases, sepsis and/or hypertension. In particular, the method is used for preoperative prediction of improvement of left ventricular heart function, e. g. following deterioration of left ventricular ejection fraction (LVEF) after acute myocardial ST-segment elevations infarction (STEMI) and coronary artery 3-vessel disease sequentially treated by acute percutaneous coronary intervention (PCI) and secondary coronary artery bypass graft (CABG) revascularization and/or ischemia-reperfusion intervention.

In yet another preferred embodiment, the method for prediction of response to cardiac regeneration, in particular to stem cell therapy and/or induction of angiogenesis response and/or tissue repair in the context of cardiovascular disease uses a sample taken from a subject suffering from heart disease and/or arteriosclerosis. Such a sample may particularly be taken from a subject suffering from angina, acute myocardial injury, cellular necrosis, myocardium hypertrophy, heart failure, preferably ischemic heart failure, non-ischemic heart failure, myocarditis, atrial fibrillation, ventricular fibrillation and/or arteriosclerosis.

According to a preferred embodiment, the method comprises profiling of the results of the comparison of at least two, three, four, five, six or more time points. Advantageously, such time points comprise a sample taken preoperative and at days 1, 3, 10, 90, 180, and 730 post operation. By analysing the samples of at least two, three, four, five, six or more time points, specificity of prediction accuracy can be beneficially increased and even more.

A further embodiment of the beneficial method comprises the use of clinical diagnostic parameters. In particular, such parameters are selected from colony forming unit (CFU) Hill assay, Matrigel Plug assay, weight, and/or left ventricular end-systolic volume (LVESV).

According to a next preferred embodiment, the method is used for profiling of angiogenesis response.

Yet another preferred embodiment includes for the advantageous method to further comprise analysing an RNA and/or DNA and/or mRNA sequence and/or functional RNA such as microRNA and/or non-coding RNA and/or SNP comprising a diagnostic signature. Within the context of the invention, such a signature analysis of an RNA and/or DNA and/or SNP may be performed and/or supported by machine learning and/or pathway analysis. In the context of the present invention, pathway analysis is used to identify related RNA and/or DNA and/or SNP within a pathway or building pathway *de novo* from the RNA, DNA and/or SNP of interest.

Also, according to a further preferred embodiment, the advantageous method may comprise analysis of pharmacokinetic and pharmacogenetic data employing RNA and/or DNA sequencing analysis and/or network pathway analysis. Such pharmacokinetic data may be in particular obtained from subjects given medications from the group selected from a Statin, acetylsalicylic acid (ASS), a β-blocker, an angiotensin-converting-enzyme (ACE) inhibitor, an angiotensin II (ATII) receptor antagonist, an Aldosterone antagonist, a diuretic agent, a Ca-antagonist, an anti-Arrhythmic agent, Digitalis, Marcumar, and/or Nitrate.

A next preferred embodiment may additionally use analysis of phenotype of the subject, such as analysis of body weight.

According to an even further preferred embodiment, the method for prediction of cardiovascular repair comprises stem cell therapy to use transplantation of CD133 positive stem cells. Such a stem cell therapy may be optionally combined with CABG revascularization, used for induction of cardiac regeneration, and/or PCI. Though, a particularly preferred embodiment includes that cardiac stem cell therapy further comprises coronary artery bypass graft surgery.

Advantageously, cardiac regeneration and/or functional improvement of the heart after cardiac stem cell therapy are accompanied by an increase of LVEF of at least 5%.

The aforementioned inventive method is not restricted to be applied for human subjects only, but may also include animals. However, it is preferred for the method to be applied to human subject/s. In accordance with the invention, a sample of such a subject may be derived from blood, in particular peripheral blood, and/or a serum and/or a plasma sample and/or a tissue biopsy sample and/or a sample of circulating (stem) cells such as endothelial progenitor cells (EPC).

As depicted above, the present invention also pertains to one or more biomarker/s selected from SH2B3-gene and/or -gene expression mutation variant and/or Lymphocyte adapter protein expression from SH2B3-gene and/or -gene expression mutation variant for use in a method for prediction of response to disease therapy, in particular to tissue regeneration, preferably to cardiovascular regeneration.

According to a preferred embodiment, the biomarker is used in combination with one or more further biomarker/s, wherein the further biomarker is selected from the group of Angiogenic factor, Survival factor, Chemokine, circulating EPC, CEC, circulating thrombocytes, circulating mononuclear cells and subpopulations, receptor/ligand expression on MNC subpopulations and/or whole genomic sequence RNA.

According to a particularly preferred embodiment, the advantageous combination further uses one or more biomarkers, wherein the further biomarker/s is/are selected from PLCG1, EPO, LPCAT2, GRB2, AP1B1, AFAP1, KLF8, MARK3, REX1BD, SACM1L, PDGFRB, VEGF, BEX3, Delta CT_SH2B3, ZNF205, LTB, EMG1, CD34+ cells/ml PB, BAZ1A and/or CD133+ cells/ml PB.

Preferably, PLCG1, LPCAT2, GRB2, AP1B1, AFAP1, KLF8, MARK3, REX1BD, SACM1L, PDGFRB, BEX3, ZNF205, EMG1, BAZ1A and/or LTB is/are determined on an RNA level and/or EPO and/or VEGF is/are determined on a protein level.

Even more preferably, determination of EPO, VEGF, Delta_CT_SH2B3, CD34+ cells/ml PB and/or CD133+ cells/ml PB is conducted from preoperative samples.

According to an even more preferred embodiment the advantageous combination of further biomarker/s is/are selected from PLCG1, EPO, LPCAT2, GRB2, AP1B1, AFAP1, KLF8, MARK3, REX1BD, SACM1L, PDGFRB and/or VEGF.

A combination of those further biomarkers may be selected on an individual basis, depending on the disease type and/or disease state of the subject or patient, respectively. For example, a selection may encompass a combination of biomarkers including PLCG1, EPO, LPCAT2, GRB2, AP1B1, KLF8, MARK3 and/or VEGF (as depicted in Fig. 3).

In addition to the above mentioned further biomarker/s, according to a next embodiment, for example, the Angiogenic factor is selected from VEGF and/or FGF, more preferably from VEGF-B and/or FGF-4, and/or HGF and/or Ang-1, the Survival factor is preferably selected from IGF-1 and the Chemokine is selected from IGF-2 and/or SDF-1.

Alternatively or additionally, such further biomarker/s comprise one or more biomarker/s selected from zinc finger protein ZDHHC2 and/or MYL4/ALC1 and/or INPP4B and/or RBM38, CD133+cells/ml PB and/or CD34+ cells/ml PB and/or CD45+184+ cells/ml PB and/orCD45+ cell/ml PB and/or CD184+ cells/ml PB and/or MNC in PB and/or CFU-Hill of PB and/or RIOK3-gene expression in PB and/or ABCC13-gene expression in PB and/or COPS3-gene expression in PB and/or GUK1-gene expression in PB and/or AP3B1-gene expression in PB and/or TBC1D22B-gene expression in PB and/or RBM38-gene expression in PB and/or ZBTB33-gene expression in PB and/or AGO2-gene expression in PB. Advantageously, the method is an ex *vivo* / *in vitro* method.

Preferably, the combination of such biomarkers is used in a method for prediction of response to disease therapy, in particular to tissue repair, preferably to cardiovascular regeneration, the method comprising
(i) determining in a sample of a subject the amount of each of the biomarkers,
(ii) comparing the determined amounts to a baseline value and/or a reference,
(iii) predicting, based on the results of the comparison, whether a response to cardiovascular repair in the subject is to be expected, not expected or is ambivalent.

According to a preferred embodiment of the present invention, a combination of the above biomarkers are used in a method for prediction of response to cardiovascular regeneration, wherein the method further comprises analysis of clinical diagnostic data, and/or RNA and/or mRNA and/or functional RNA such as microRNA and/or non-coding RNA and/or SNP, and/or analysis of pharmacokinetic data, and/or analysis of phenotyping, e.g. body weight.

Yet more preferably, an advantageous method and/or an advantageous combination of the above biomarkers is/are used for preoperative prediction of response to stem cell therapy and wherein the stem cell therapy is accompanied by CABG.

The term "stem cell therapy" as used herein refers to all therapeutic approaches which comprise the transplantation of exogenous cardiomyocytes into the heart of a subject to be treated. Such cardiomyocytes may be generated by reprogramming non-cardiomyocyte progenitor cells into cardiomyocytes. Cells to be reprogrammed may be embryonic stem cells, induced pluripotent stem cells, multipotent cardiac progenitor cells, skeletal myoblasts or bone marrow derived stem cells. Moreover, mature cardiomyocytes may also be used, in particular, if stimulated to reenter into mitotic cell cycling. More preferably, the cardiac stem cell therapy according to the present invention comprises transplantation of CD133-positive cells, most preferably, CD133-positive bone marrow mononuclear cells. The cells may be transplanted as isolated single cells or in a preformed arrangement such as a tissue block formed by tissue engineering processes. Preferably, the cells are transplanted in accordance with the present invention by intra-myocardial injection. Moreover, the term cardiac stem cell therapy may also encompass additional therapeutic measures such as drug treatments accompanying the transplantation process or other therapeutic measures such as surgery. Preferably, the cardiac stem cell therapy in accordance with the present invention further comprises coronary artery bypass graft surgery as described in the accompanying examples below.

The term "functional improvement of the heart" as used herein refers to a significant increase in the LVEF of the heart observed when comparing said LVEF before and after treatment of the subject. Preferably, a significant increase is an increase of 5% or more of LVEF observed after treatment. An increase of less than 5% is deemed to be insignificant. Further parameters which may be considered in addition for finding a functional improvement are a more than 10% decrease in perfusion defect size, a more than 10% decrease in left ventricle end systolic volume (LVESV) as quantified by MIBI SPECT and a more than 10% increase in peak systolic velocity measured by transthoracic echocardiogram.

Heart failure as used herein refers to any functional impairment of the heart including left-sided failures, right-sided failures or biventricular failures. Typically, the term heart failure as referred to herein is left-sided failure that results in reduced ejection fractions, e.g. a significantly reduced LVEF. Further symptoms of heart failure are well known to the clinician. Heart failure as referred to herein encompasses acute and chronic forms of heart failure and any stage of severity, e.g. for left-sided failures all stages according to the New York Heart Association (NYHA) classification system, NYHA I to IV.

The term "PLCG1" as used herein refers to a protein encoded by the corresponding gene and catalyzes the formation of inositol 1,4,5-trisphosphate and diacylglycerol from phosphatidylinositol 4,5-bisphosphate. This reaction plays an important role in the intracellular transduction of receptor-mediated tyrosine kinase activators. Two transcript variants encoding different isoforms have been found for this gene.

The term "LPCAT2" as used herein refers to a gene that encodes a member of the lysophospholipid acyltransferase family. The encoded protein may function in membrane biogenesis and production of platelet-activating factor in inflammatory cells. The enzyme may localize to the endoplasmic reticulum and the Golgi.

The term "GRB2" as used herein refers to Growth factor receptor-bound protein 2, also known as Grb2 and is an adaptor protein involved in signal transduction/cell communication. In humans, the GRB2 protein is encoded by the GRB2 gene. The protein encoded by this gene binds receptors such as the epidermal growth factor receptor and contains one SH2 domain and two SH3 domains.

The term "AP1B1" as used herein rerfers to AP-1 complex subunit beta-1 and is a protein that in humans is encoded by the AP1 B1 gene . Adaptor protein complex 1 is found at the cytoplasmic face of coated vesicles located at the Golgi complex, where it mediates both the recruitment of clathrin to the membrane and the recognition of sorting signals within the cytosolic tails of transmembrane receptors. This complex is a heterotetramer composed of two large, one medium, and one small adaptin subunit. The protein encoded by this gene serves as one of the large subunits of this complex and is a member of the adaptin protein family.

The term "AFAP1" as used herein refers to Actin filament-associated protein 1 is a protein that in humans is encoded by the AFAP1 gene . The protein encoded by this gene is a Src binding partner. It may represent a potential modulator of actin filament integrity in response to cellular signals, and may function as an adaptor protein by linking Src family members and/or other signaling proteins to actin filaments. Two alternative transcripts encoding the same protein have been identified.

The term "KLF8" as used herein refers to Krueppel-like factor 8 and is a protein that in humans is encoded by the KLF8 gene. KLF8 belongs to the family of KLF protein. KLF8 is activated by KLF1 along with KLF3 while KLF3 represses KLF8.

The term "MARK3" as used herein refers to MAP/microtubule affinity-regulating kinase 3 and is an enzyme that in humans is encoded by the MARK3 gene. MARK3 has been shown to interact with stratifin.

The term "REX1BD" as used herein refers to required for excision 1-B domain containing' and is a protein coding gene. It interacts with 3-isobutyl-1-methyl-7H-xanthine, aflatoxin B1 and aflatoxin B2.

The term "SACM1L" as used herein refers to Staphylococcus aureus Cowan 1 phosphatidylinositide phosphatase and is an enzyme that in humans is encoded by the SACM1L gene.

The term "PDGFRB" as used herein refers to PDGFRB gene and encodes a typical receptor tyrosine kinase, which belongs to the type III tyrosine kinase receptor (RTK) family and is structurally characterized by five extracellular immunoglobulin-like domains, a single membrane-spanning helix domain, an intracellular juxtamembrane domain, a split tyrosine kinase domain and a carboxylic tail. Upon PDGF binding the dimerization of receptor releases the inhibitory conformations due to auto-phosphorylation of regulatory tyrosine residues in trans fashion. Tyrosine residues 857 and 751 are major phosphorylation sites for the activation of PDGFRβ.

The term "BEX3" as used herein refers to Brain Expressed X-Linked 3 and is a protein coding gene. Among its related pathways are p75 NTR receptor-mediated signalling and signalling by GPCR.

The term "ZNF205" as used herein refers to Zinc Finger Protein 205 and is a protein coding gene. Gene Ontology (GO) annotations related to this gene include nucleic acid binding and DNA-binding transcription factor activity.

The term "LTB" refers to Lymphotoxin Beta and is a protein coding gene. Among its related pathways are innate immune system and innate lymphoid cell differentiation pathways. Gene Ontology (GO) annotations related to this gene include signaling receptor binding and tumor necrosis factor receptor binding.

The term "EMG1" as used herein refers to EMG1 N1-Specific Pseudouridine Methyltransferase and is a protein coding gene. Among its related pathways are rRNA processing in the nucleus and cytosol and ribosome biogenesis in eukaryotes. Gene Ontology (GO) annotations related to this gene include methyltransferase activity.

The term "BAZ1A" as used herein refers to Bromodomain adjacent to Zinc Finger Domain 1A and is a component of the histone-fold protein CHRAC complex which facilitates nucleosome sliding by the ACF complex and enhances ACF-mediated chromatin assembly. The C-terminal regions of both CHRAC1 and POLE1 are required for these functions.

The term "vascular endothelial growth factor (VEGF)" as used herein refers to soluble polypeptide growth factor which stimulates angiogenesis, vasculogenesis and vascular permeability. It is produced by various cell types. There are five different VEGF polypeptides, VEGF-A, placenta growth factor (PGF), VEGF-B, VEGF-C, and VEGF-D. Preferably, the VEGF is selected from VEGF-B. In contrast to VEGF-A, VEGF-B plays a less pronounced role in the vascular system. Whereas VEGF-A is important for the formation of blood vessels, such as during development or in pathological conditions, VEGF-B seems to play a role only in the maintenance of newly formed blood vessels during pathological conditions. VEGF-B plays also an important role on several types of neurons. It is important for the protection of neurons in the retina and the cerebral cortex during stroke and of motoneurons during motor neuron diseases such as amyotrophic lateral sclerosis. VEGF-B exerts its effects via the FLT1 receptor (also known as Vascular Endothelial Growth Factor Receptor 1). VEGF-B has also been found to control endothelial uptake and transport of fatty acids in heart and skeletal muscle.

The term "Fibroblast growth factor (FGF)" as used herein refers to a family of growth factors, with members involved in angiogenesis, wound healing, embryonic development and various endocrine signaling pathways. Preferably, the FGF is selected from FGF-4. The term "FGF-4" as used herein refers to Fibroblast growth factor 4 protein that in humans is encoded by the FGF4-gene.

The term "HGF" as used herein refers to Hepatocyte growth factor (HGF) or scatter factor (SF), which is a paracrine cellular growth, motility and morphogenic factor. It is secreted by mesenchymal cells and targets and acts primarily upon epithelial cells and endothelial cells, but also acts on hematopoietic progenitor cells and T cells.

The term "Ang-1" as used herein refers to Angiopoietin 1, which is a type of Angiopoietin and is encoded by the gene ANGPT1. Angiopoietin is part of a family of vascular growth factors that play a role in embryonic and postnatal angiogenesis. Angiopoietin signaling most directly corresponds with angiogenesis, the process by which new arteries and veins form from preexisting blood vessels. Angiogenesis proceeds through sprouting, endothelial cell migration, proliferation, and vessel destabilization and stabilization. They are responsible for assembling and disassembling the endothelial lining of blood vessels. Angiopoietin Cytokines are involved with controlling microvascular permeability, vasodilation, and vasoconstriction by signaling smooth muscle cell surrounding vessels.

The term "Survival factor" as used herein encompasses a group of substances exhibiting anti-apoptotic effect.

The term "IGF-1" (Insulin like growth factor 1), also called Somatomedin C, refers to a protein that in humans is encoded by the IGF-1-gene. IGF-1 is a hormone similar in molecular structure to Insulin. It plays an important role in childhood growth and continues to have anabolic effects in adults. IGF-1 consists of 70 amino acids in a single chain with three intramolecular disulfide bridges. IGF-1 has a molecular weight of 7,649 Daltons.

The term "Chemokine" as used herein refers to a family of small cytokines or signaling proteins secreted by cells. Their name is derived from their ability to induce directed chemotaxis in nearby responsive cells; they are chemotactic cytokines.

Some chemokines are considered pro-inflammatory and can be induced during an immune response to recruit cells of the immune system to a site of infection, while others are considered homeostatic and are involved in controlling the migration of cells during normal processes of tissue maintenance or development. Chemokines are found in all vertebrates, some viruses and some bacteria, but none have been described for other invertebrates.

Chemokines have been classified into four main subfamilies: CXC, CC, CX3C and XC. All of these proteins exert their biological effects by interacting with G protein-linked transmembrane receptors called chemokine receptors that are selectively found on the surfaces of their target cells.

The term "Erythropoietin (EPO)" as used herein refers to a soluble polypeptide being a cytokine. EPO is produced by kidney cells, typically, under hypoxic conditions. The term also encompasses variants of the human EPO polypeptides. Such variants have at least the same essential biological and immunological properties as the aforementioned EPO polypeptide. In particular, they share the same essential biological and immunological properties if they are detectable by the same specific assays referred to in this specification, e.g., by ELISA assays using polyclonal or monoclonal antibodies specifically recognizing the said EPO polypeptides. Moreover, it is to be understood that a variant as referred to in accordance with the present invention may have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific IL-6. Variants may be allelic variants, splice variants or any other species specific homologs, paralogs, or orthologs. Moreover, the variants referred to herein include fragments of the specific EPO or the aforementioned types of variants as long as these fragments have the essential immunological and biological properties as referred to above. Such fragments may be, e.g., degradation products of EPO. Variants are deemed to share the same essential biological and immunological properties if they are detectable by the same specific assays referred to in this specification, e.g., by ELISA assays using polyclonal or monoclonal antibodies specifically recognizing the said EPO polypeptides. A preferred assay is described in the accompanying Examples. Further included are variants which differ due to posttranslational modifications such as phosphorylation or myristylation.

The term "SH2B adapter protein 3 (SH2B3)" as used herein refers to lymphocyte adapter protein (LNK). SH2B3 is a protein that in humans is encoded by the SH2B3-gene on chromosome 12. It is ubiquitously expressed in many tissues and cell types (Li Y, He X, Schembri-King J, Jakes S, Hayashi J, May 2000. Journal of Immunology. 164(10):5199-206).

The term "Interleukin-6 (IL-6)" as used herein refers to a soluble polypeptide being a pro-inflammatory cytokine and anti-inflammatory myokine. It is produced by T-cells and macrophages.

Preferably, IL-6 refers to human IL-6 as described, e. g., in Wong 1988, Behring Inst. Mitt 83: 40-47. More preferably, human IL-6 has an amino acid sequence as shown in Genbank accession number p05231.1, GI: 124347. The term also encompasses variants of the aforementioned human IL-6 polypeptides. Such variants have at least the same essential biological and immunological properties as the aforementioned IL-6 polypeptide. In particular, they share the same essential biological and immunological properties if they are detectable by the same specific assays referred to in this specification, e.g., by ELISA assays using polyclonal or monoclonal antibodies specifically recognizing the said IL-6 polypeptides. Moreover, it is to be understood that a variant as referred to in accordance with the present invention may have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific IL-6. Variants may be allelic variants, splice variants or any other species specific homologs, paralogs, or orthologs. Moreover, the variants referred to herein include fragments of the specific IL-6 or the aforementioned types of variants as long as these fragments have the essential immunological and biological properties as referred to above. Such fragments may be, e.g., degradation products of IL-6. Variants are deemed to share the same essential biological and immunological properties if they are detectable by the same specific assays referred to in this specification, e.g., by ELISA assays using polyclonal or monoclonal antibodies specifically recognizing the said IL-6 polypeptides. A preferred assay is described in the accompanying Examples. Further included are variants which differ due to posttranslational modifications such as phosphorylation or myristylation.

The term "insulin-like growth factor(IGF-2)" as used herein refers to one of three protein hormones that share structural similarity to insulin. IFG-2 is believed to be secreted by the liver and to circulate in the blood. It has growth-regulating, insulin-like and mitogenic activities. The growth factor has a major, but not absolute, dependence on somatotropin. It is believed to be a major fetal growth factor.

The term "stomal cell-derived factor 1 (SDF-1)", also known as C-X-C motif chemokine 12 (CXCL12), is a chemokine protein that in humans is encoded by the CXCL12-gene on chromosome 10. It is ubiquitously expressed in many tissues and cell types. Stromal cell-derived factors 1-alpha and 1-beta are small cytokines that belong to the chemokine family, members of which activate leukocytes and are often induced by pro-inflammatory stimuli such as lipopolysaccharide, tumor necrosis factor (TNF), or IL1. The chemokines are characterized by the presence of four conserved cysteines that form two disulfide bonds.

The term "zinc finger proteins (ZNFs)" as used herein refers to proteins and have a wide range of molecular functions. Given the wide variety of zinc-finger domains, ZNFs are able to interact with DNA, RNA, PAR (poly-ADP-ribose) and other proteins. Thus, ZNFs are involved in the regulation of several cellular processes. ZNFs are implicated in transcriptional regulation, ubiquitin-mediated protein degradation, signal transduction, actin targeting, DNA repair, cell migration, and numerous other processes. The term "ZDHHC2" as used herein refers to zinc fingers DHHC type.

The term "MYL4/ALC1" as used herein refers to Atrial Light Chain-1 (ALC-1), also known as Essential Light Chain. ALC-1 is a protein that in humans is encoded by the MYL4 gene.

The term "INPP4B" as used herein refers to Inositol polyphosphate 4-phosphatase II, which is a regulator of the phosphoinositide 3-kinase (PI3K) signaling pathway and is implicated as a tumor suppressor in epithelial carcinomas.

The term "RBM38" refers to RNA binding motif protein 38. RBM38, also named RNPC1, induces cell-cycle arrest in G1, at least in part, via binding to and stabilizing the mRNA of the cyclin-dependent kinase inhibitor p21.

The term "MNC" as used herein refers to mononuclear cells, which is any cell that has a single round nucleus; examples of a mononuclear cell are lymphocytes, monocytes, and dendritic cells. These cells are critical components of the immune system, which are involved in both humoral and cell-mediated immunity. MNCs are widely used product in research and clinical applications such as in microbiology, virology, oncology, vaccine development, transplant and regenerative biology, and toxicology.

The term "CFU-Hill" as used herein refers to an early outgrowth, formed by plating peripheral blood mononuclear cells on fibronectin-coated dishes, allowing adhesion and depleting non-adherent cells, and isolating discrete colonies.

The term "ABCC13" as used herein refers to ATP-binding cassette transporter sub-family C member 13, which is a protein that in humans is encoded by the ABCC13 gene.

The term "COPS3" as used herein refers to a protein that in human is encoded by the COPS3-gene. It encodes a subunit of the COP9 signalosome. The protein complex is supposed to exhibit isopeptidase activity. Among its related pathways are transcription-coupled nucleotide excision repair (TC-NER) and clathrin-mediated endocytosis. It is found in eukaryotic organisms including human.

The term "GUK1" as used herein refers to Guanylate kinase and is an enzyme that in humans is encoded by the GUK1 gene.

The term "AP3B1" as used herein refers to AP-3 complex subunit beta-1 which is a protein that in humans is encoded by the AP3B1 gene. This gene encodes a protein that may play a role in organelle biogenesis associated with melanosomes, platelet dense granules, and lysosomes. The encoded protein is part of the heterotetrameric AP-3 protein complex which interacts with the scaffolding protein clathrin.

The term "TBC1D22B" as used herein refers to TBC1D22B (TBC1 Domain Family Member 22B), which is a protein coding gene and may act as a GTPase-activating protein for Rab (Ras-ralated protein) family protein(s). Gene Ontology (GO) annotations related to this gene include GTPase activator activity. A paralog of this gene is TBC1 D22A.

The term "ZBTB33" as used herein refers to Transcriptional regulator Kaiso, which is a protein that in humans is encoded by the ZBTB33-gene. This gene encodes a transcriptional regulator with bimodal DNA-binding specificity, which binds to methylated CGCG and also to the non-methylated consensus KAISO-binding site TCCTGCNA. The protein contains an N-terminal POZ/BTB domain and 3 C-terminal zinc finger motifs. It recruits the N-CoR repressor complex to promote histone deacetylation and the formation of repressive chromatin structures in target gene promoters. It may contribute to the repression of target genes of the Wnt (the term "Wnt" encompasses a diverse family of secreted lipid-modified signaling glycoproteins that are 350-400 amino acids in length) signaling pathway, and may also activate transcription of a subset of target genes by the recruitment of catenin delta-2 (CTNND2). Its interaction with catenin delta-1 (CTNND1) inhibits binding to both methylated and non-methylated DNA. It also interacts directly with the nuclear import receptor Importin-α2 (also known as karyopherin alpha2 or RAG cohort 1), which may mediate nuclear import of this protein. Alternatively spliced transcript variants encoding the same protein have been identified.

The term "AGO2" as used herein refers to a member of the Argonaute protein family which plays a central role in RNA silencing processes, as essential components of the RNA-induced silencing complex (RISC). Endonuclease activity and thus RNAi-dependent gene silencing exclusively belongs to AGO2. Considering the sequence conservation of PAZ and PIWI domains across the family, the uniqueness of AGO2 is presumed to arise from either the N-terminus or the spacing region linking PAZ and PIWI motifs.

Determining the amount of a biomarker may, preferably, comprise the steps of (a) contacting the biomarker with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide or protein or nucleic acid or cell described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analysed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Further suitable techniques for the determination of a biomarker are described in the following.

First, binding of a ligand may be measured directly, e.g. by NMR or surface plasmon resonance. Second, if the ligand also serves as a substrate of an enzymatic activity of the biomarker of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the biomarker, respectively, may be contacted with a suitable substrate allowing detection by the generation of an intensity signal. For measurement of enzymatic reaction products, preferably the amount of substrate is saturating. The substrate may also be labelled with a detectable label prior to the reaction. Preferably, the sample is contacted with the substrate for an adequate period of time. An adequate period of time refers to the time necessary for a detectable, preferably measurable, amount of product to be produced. Instead of measuring the amount of product, the time necessary for appearance of a given (e.g. detectable) amount of product can be measured. Third, the ligand may be coupled covalently or non-covalently to a label allowing detection and measurement of the ligand. Labelling may be done by direct or indirect methods. Direct labelling involves coupling of the label directly (covalently or non-covalently) to the ligand. Indirect labelling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system. The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridin ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate. A suitable enzyme-substrate combination may result in a coloured reaction product, fluorescence or chemoluminescence, which can be measured according to methods known in the art (e.g. using a light-sensitive film or a suitable camera system). As for measuring the enzymatic reaction, the criteria given above apply analogously. Typical fluorescent labels include fluorescent proteins (such as GFP and its derivatives), Cy3, Cy5, Texas Red, Fluorescein, and the Alexa dyes. Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager.

The amount of a biomarker may be, also preferably, determined as follows: (a) contacting a solid support comprising a ligand for the biomarker as specified above with a sample comprising the biomarker and (b) measuring the amount of biomarker which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand/s are well known and include, but are not limited to ionic, hydrophobic or covalent interactions and the like.

Suitable measurement methods according to the present invention also include fluorescence activated cell sorting (FACS) analysis, radioimmunoassay (RIA), enzyme-linked immunosorbent assay (ELISA), sandwich enzyme immune tests, electrochemiluminescence sandwich immunoassays (ECLIA), dissociation-enhanced lanthanide fluoro immuno assay (DELFIA), scintillation proximity assay (SPA) or solid phase immune tests. Further methods known in the art (such as gel electrophoresis, 2D gel electrophoresis, SDS polyacrylamid gel electrophoresis (SDS-PAGE), Western Blotting and mass spectrometry (MS) can be used alone or in combination with labelling or other detection methods as described above.

In order to carry out the method of the present invention, a kit is provided which is adapted for carrying out the inventive method, wherein the kit comprises detection agents for determining in a sample of said subject the amount of at least one of the above biomarkers. Such a kit advantageously allows for a straight forward conduction of the inventive method and/or measurement and/or determination of the biomarker/s according to the present invention.

The term "kit" as used herein refers to a collection of the aforementioned components, preferably, the components being provided separately or within a single container. The kit may also comprise instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as a storage medium (e.g. a Compact Disc, USB drives or external hard disks) or directly on a computer or data processing device. Moreover, the kit may, preferably, comprise standards for reference amounts as described elsewhere herein in detail.

Further characteristics of the present invention are derived from the examples in combination with the claims and the figures. Single features may be, in a particular embodiment, realised in combination with other features and do not limit the scope of protection of the present invention. The following description of the examples according to the invention may relate to the figures, whereby
**Figure 1** depicts a targeted DNAseq and RNAseq variant summary of SH2B3 according to the present invention. The plot shows the ratio of SNP / deletion sites that are identified in Responders (first percentage in brackets) and Non-responders (second percentage in brackets) as well as the possible amino acid transfer.

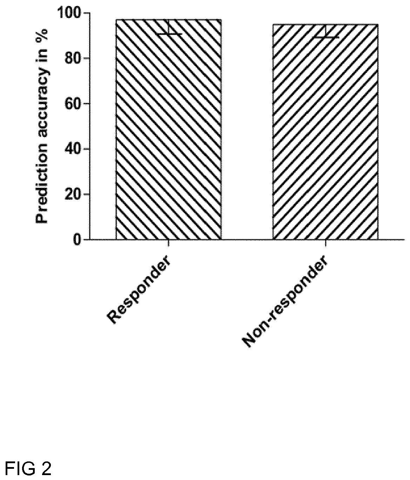

**Figure 2** depicts Machine learning accuracy comparison for the supervised prediction of the patient responsiveness using only preoperative data. Results are obtained after feature selection and subsequent prediction with two independent classifiers. The graphs show the true positive prediction results of two ML models (AdaBoost for feature selection and RF and SVM for the study). The error bars indicate the respective accuracy standard deviation for the constructed models that have been obtained after 100 iterations.
**Figure 3** shows the top twenty biomarkers obtained from machine learning feature selection based on the Random forest classifier. The underlying initial data was the PERFECT trial accompanying research data, patient sequencing data (esp. differentially expressed transcripts, co-expressed transcripts, and transcripts correlated with SH2B3 in time) as well as RT-PCR data. Combinations and subsets of these twenty features have been subsequently used to train the final model, which includes eight features marked in dotted lines. The importance indicates a hierarchy of the most relevant features needed for a classification.

### EXAMPLES

The following examples shall merely illustrate the invention. They shall, whatsoever, not be construed as limiting the scope thereof.

### Example 1: Clinical study design and evaluation

Peripheral blood bone marrow response was studied by whole genome and circulating EPC analysis in the randomized phase 3 PERFECT trial biomarker subgroup at the Rostock trial site with complete available biobank, clinical (per protocol), and biomarker data (n=23) (Steinhoff G, Nesteruk J, Wolfien M, et al. EBioMedicine 2017 Aug; 22:208-224. doi: 10.1016/j.ebiom.2017.07.022. Epub 2017 Jul 29.). CD133⁺ BMSC treated (n=13) and placebo controls (n=14) were equally distributed. In the biomarker patient cohort (n=23; Placebo/CD133⁺9/14) of the PERFECT trial we investigated systemic bone marrow stem cell response in peripheral blood in Responders (R) classified by ΔLVEF≥5 % after 180 d (n=14; Placebo/CD133⁺ 7/7) and Non-responders (NR) classified by ΔLVEF<5 % after 180 d (n=9; Placebo/CD133⁺ 5/4) in both treatment groups (intramyocardial Placebo vs. CD133+).

The R/NR gene expression and biomarker data of postischemic myocardial regeneration were compared to mouse Lnk/SH2B3 knockout deficient conditions studied in an experimental setting of myocardial infarction in Lnk^{-/-} mice vs. Lnk^{+/+} wild type (WT) mice studied at different time points (day 0, 1, 3, 7, 14, 28) before and post-MI.

### Clinical trial setting

The PERFECT trial was a randomised, multicenter, placebo-controlled, double-blinded phase 3 study investigating the myocardial regeneration effects of intramyocardial CD133⁺ BMSC treatment in combination with coronary artery bypass graft (CABG) revascularization for post infarction myocardial ischemia (Steinhoff G, Nesteruk J, Wolfien M, et al. EBioMedicine 2017 Aug; 22:208-224. doi: 10.1016/j.ebiom.2017.07.022. Epub 2017 Jul 29., Baughn LB, Meredith MM, Oseth L, Smolarek TA, Hirsch B. Cancer Genet. 2018 Oct; 226-227:30-35. doi: 10.1016/j.cancergen.2018.05.004. Epub 2018 Jun 8.). A prespecified and post-hoc biomarker outcome analysis was published (Steinhoff G, Nesteruk J, Wolfien M, et al. EBioMedicine 2017; Aug;22:208-224. doi: 10.1016/j.ebiom.2017.07.022. Epub 2017 Jul 29.). Inclusion criteria of the PERFECT trial were (a) coronary artery disease after MI with the indication for CABG surgery, (b) reduced LVEF (25-50 %) and (c) presence of a localized kinetic/hypokinetic/hypoperfused area of left ventricular (LV) myocardium defining the SC target area. Assessments were performed preoperative and at days 1, 3, 10, 90, 180, and 730 post operation.

### Post Hoc Gene Expression Analysis

RNAseq analysis and mRNA RT-PCR in PB: Analyses and examinations were performed before unblinding of the trial and under careful adherence to the protection of data privacy (pseudonyms).

### Transcriptome Analysis of EDTA blood samples using NGS (Next Generation Sequencing)

RNA of frozen EDTA blood samples was isolated in a three step procedure: First, the GeneJET Stabilized and Fresh Whole Blood RNA Kit (Thermo Scientific) was used following manufacturer's instructions. Second, isolated RNA was precipitated with 2,5 volumes ethanol under high salt conditions (10 % of 3 M sodium acetate, pH 5.2). After DNase digest (Thermo Scientific) the RNA was finally purified using Agencourt RNAClean XP beads (Beckman Coulter). Isolated RNA was analyzed on a Bionalyzer (Agilent) using RNA 6000 Nano Chips (Agilent). Quality controlled RNA was used to construct sequencing libraries using the Universal Plus mRNA-Seq Technology (Nugen) following manufacturer's instructions. Brief: mRNA was selected by oligo d(T) beads, reverse transcribed and Globin derived cDNA was cleaved by the Globin depletion module (Nugen). Quality controlled and quantified libraries were sequenced on a HiSeq1500 system (Illumina) in single-end mode (100 nt read length). For RNAseq data analysis, we performed adapter clipping and quality trimming procedures for data pre-processing and aligned the reads to the hg19 genome with the aid of kallisto. Differential expression analysis was performed using the likelihood ratio test of the sleuth package (genes with >2-fold change and a q value < 0.05 are considered as significantly differentially expressed). The gene annotation, including functional annotation clustering and functional classification, was performed with Enrichr (Kuleshov, M. V. et al., Nucleic Acids Res. 2016, vol 44(W1), doi: 10.1093/nar/gkw377, Epub 2016 May 3).

### Variant calling from transcriptomic data

The previously preprocessed RNAseq datasets (preoperative and postoperative) have been aligned to the hg19 reference with Star (2-pass mode). The variant calling was applied by the Gatk toolkit (McKenna, A. et al., Genome Res. 2010, vol. 20(9): 1297-1303, doi: 10.1101/gr.107524.110) with specialized filters (e.g. variants are only considered, if they are confirmed with five independent reads). Variant annotation was done with reftools.

### Experimental Lnk/SH2B3^{-/-} mouse model

The *Lnk*/*SH2B3*^{*-*/*-*} mouse strain was generated as described previously (Takaki S. et al., J. Exp. Med. 2002; 195:151-160). C57BL/6 mice (CLEA Japan, Tokyo, Japan) were used as WT control mice. GFP transgenic mice (GFP-Tg mice; C57BL/ 6TgN [act EGFP] Osb Y01) were mated with WT mice or *Lnk*/*SH2B3*^{*-*/*-*} mice and generated *WT*/*GFP* mice or *Lnk*/*SH2B3*^{*-*/*-*}/*GFP* mice, respectively, for BM transplantation (BMT) studies. All experimental procedures were conducted in accordance with the Japanese Physiological Society Guidelines for the Care and Use of Laboratory Animals and the study protocol was approved by the Ethics Committee in RIKEN Center for Developmental Biology.

### Induction of myocardial infarction (MI)

Eight-week-old to twelve-week-old mice were anesthetized with an intraperitoneal injection of 400 mg/kg 2,2,2-tribromoethanol (Avertin; Sigma, St.Louis, MO). MI was induced by ligating left arterior descending (LAD) coronary artery as described previously (Fabregat A, Sidiropoulos K, Garapati P, et al. Nucleic Acids Res 2016, Jan 4; 44 (D1):D481-7. doi: 10.1093/nar/gkv1351. Epub 2015 Dec 9.).

### Tissue harvesting

At necropsy, hearts were embedded in O.C.T.^{™} Compound (Tissue-Tek^{®}) followed by snap frozen in liquid nitrogen, and sectioned at 6 µm by a cryostat (Leica microsystems, Wetzlar, Germany). Total RNA for RT-PCR analysis was isolated by selective dissection of fibrosis and peri-infarction area in LV myocardium. For BrdU incorporation study, mice were injected 100 µL of 10 mg/mL BrdU solution (BD Pharmingen) intraperitoneally 16 hours before sacrifice.

### RT-PCR analyses

Total RNA was obtained from mouse heart tissue at pre- and 3 days post MI using a TRIzol (Invitrogen) according to the manufacturer's instructions. The first-strand cDNA was synthesized using the PrimeScript RT reagent Kit (Takara Bio, Otsu, Japan) and amplified by AmpliTaq Gold DNA polymerase (Applied Biosystems, Foster City, CA). PCR was performed using a PCR thermal cycler (MJ Research PTC-225, Bio-Rad, Hercules, CA). The mouse Lnk/SH2B3 and β-actin were amplified by the following conditions: Lnk/SH2B3, 42 cycles of 94 °C for 30 seconds, 57 °C for 30 seconds, and 72 °C for 30 seconds, followed by a final hold at 72 °C for 5 minutes; β-actin, 35 cycles of 94 °C for 30 seconds, 57 °C for 30 seconds, and 72 °C for 30 seconds, followed by a final hold at 72 °C for 5 minutes. Subsequently, PCR products were visualized in 1.5 % ethidium bromide-stained agarose gels with a 100-bp DNA Ladder (Invitrogen). Primer sequences are indicated in Supplemental Table 1S.

### Quantitative real-time RT-PCR analyses

Total RNA was obtained from KSL cells using RNeasy Mini Kit (QIAGEN, Hilden, Germany) according to the manufacturer's procedure. After the first-strand cDNA was synthesized, real-time quantitative RT-PCR was performed with ABI Prism 7700 (Applied Biosystems) using SYBR Green Master Mix Reagent (Applied Biosystems). Primer sequences are indicated in Supplemental Table 1S.

### Statistical Analysis

The results were statistically analyzed using a software package (Statview 5.0, Abacus Concepts Inc, Berkeley, CA). All values were expressed as mean±standard deviation (mean±SD). The comparisons among more than three groups were made using the one-way analysis of variances (ANOVA) in Prism 4 (GraphPad Software, San Diego, CA). Post hoc analysis was performed by Tukey's multiple comparison test. Differences of P<0.05 were considered to denote statistical significance.

### Data Analysis with Machine Learning

Identifying key features and classification of the comprehensive patient data was obtained by employing supervised and unsupervised ML algorithms (Steinhoff G, Nesteruk J, Wolfien M, et al. EBioMedicine 2017 Aug; 22:208-224. doi: 10.1016/j.ebiom.2017.07.022. Epub 2017 Jul 29.). We preprocessed the data while removing features with low variance and high correlation for dimension reduction following best practices recommendations. We compared the following supervised algorithms: AdaBoost, Gradient Boosting (GB), Support Vector Machines (SVM) and Random Forest (RF) (Steinhoff G, Nesteruk J, Wolfien M, et al. EBioMedicine 2017 Aug; 22:208-224. doi: 10.1016/j.ebiom.2017.07.022. Epub 2017 Jul 29.). We employed classifiers that are suitable for training on small data sets for a comparison of features given little training and chose the most appropriate algorithm according to accuracy and robustness towards overfitting (Al-Naqeb D. Scientifica (Cairo). 2016; 2016:2079704. doi: 10.1155/2016/2079704. Epub 2016 May 30.). Supervised ML models have been 10-fold cross-validated and 100 times repeated. We then applied feature selection for the AdaBoost, GB and RF classifiers to further reduce the number of features to <20. We employed t-distributed stochastic neighbor embedding (t-SNE) for unsupervised machine learning classification and nonlinear dimensionality reduction (Steinhoff G, Nesteruk J, Wolfien M, et al. EBioMedicine 2017 Aug; 22:208-224. doi: 10.1016/j.ebiom.2017.07.022. Epub 2017 Jul 29.).

### Time course network enrichment

By the means of Systems Biology approaches, we aimed to uncover time-dependent expression response patterns among the investigated patient datasets. The identification of enriched signaling pathways was done by using the Reactome Functional Interaction (RFI) and the BisoGenenet Cytoscape applications, which were designed to find enhanced pathways and network patterns within curated and experimentally validated databases (Fabregat A, Sidiropoulos K, Garapati P, et al. Nucleic Acids Res 2016; Jan 4; 44 D1:D481-7. doi: 10.1093/nar/gkv1351. Epub 2015 Dec 9.). In addition, we subsequently used TiCoNE (Time Course Network Enricher) for the time course network enrichment analysis of the patient-specific expression data. The tool employs different mathematical algorithms to identify time patterns emerging in the expression data for a given interaction network by using the partitioning around medoids (PAM) and the Clustering for Large Applications (CLARA) algorithm, which are a specialized *k*-medoid clustering approaches. The error rates (FDR) and p-values have been computed by using globally permutations with 1000 iterations. The Pearson Product Moment Correlation (PPMC) has been used to compare the different clusters. The gene expression patterns within the motifs are considered to be common for p-values < 0.05.

Weighted gene coexpression network analysis (WGCNA) was performed by applying the R package "WGCNA" to RNAseq data. We first constructed the topological overlap matrix (TOM) of all investigated transcripts (~160,000) using the soft thresholding method. We calculated the eigenvalues of the transcripts and evaluated the adjacency based on distance. We subjected transcripts to hierarchical clustering (average linkage) and assigned transcripts with the dynamic hybrid method into groups. We computed the connectivity based on the interaction partners (*k*) and evaluated the gene significance, which represents the resulting module membership.

### Extended gene expression analysis and validation in 37 patients

Peripheral blood (PB) whole genome expression and circulating endothelial progenitor cells (EPC) analysis were studied at day 0,1,3,10 in the phase 3 PERFECT (CABG and CD133⁺BMSC or Placebo) trial biomarker subgroup (n = 23) of responders (R, n = 14; ΔLVEF +16% day 180/0) and non-responders (NR, n = 9; ΔLVEF -1,1% day 180/0) and in independent center biomarker patient cohort (n = 14).

Left ventricular functional recovery was accompanied by an increase in myocardial perfusion (R vs NR, p<0,05). Twenty biomarkers were identified for preoperative prediction of cardiac regeneration capacity (R/NR 95,6 % accuracy, 91% AUC) in PB (Figure 3). These were identified based on a ML clustering analysis that revealed three distinct gene profile subgroups in which the preoperative gene-expression signature in R differed from NR in a total of 700 genes in differential expression (n = 23, q < 0,05). Likewise the identified SH2B3 related coexpression hub genes NOTCH2, MTOR, KIT contained variants associated to the respective NR/R subgroups were mainly correlated to myocardial perfusion, ΔLVEF, PB-CD133+EPC and ΔCT SH2B3 (p<0,01).

### Abbreviation Code

ACE = Angiotensin Converting Enzyme
AE = Adverse Event
AESI = Adverse Event of Special Interest
AFAP1 = Actin Filament Associated Protein 1
AHA = American Heart Association
ANCOVA = Analysis of Covariance
ANOVA = Analysis of Variances
Ang-1 = Angiopoietin 1
AP1B1 = Adaptor Related Protein Complex 1 Subunit Beta 1
ASS = Acetylsalicylic Acid
ATII = Angiotensin II
AUC = Area Under the Curve
BAZ1A = Bromodomain adjacent to zinc finger domain protein 1A
BCIP = 5-Bromo-4chloro-3-indolyl-phosphate
BEX3 = Brain Expressed X- Linked 3
BM = Bone Marrow
BMSC = Bone Marrow Stem Cells
CABG = Coronary Artery Bypass Graft
CAP-EPC = Concentrated Ambient Particles - Endothelial Progenitor Cells
CBA = Cytometric Bead Array
CCS = Canadian Cardiovascular Society
CCTRN = Cardiovascular Cell Therapy Research Network
CD = Cluster of Differentiation
CEC = Circulating endothelial cells, CEC panel, CDs measured in PB
CFU = Colony-forming unit
CI = Confidence interval
CMV = Cytomegalovirus
CXCL12 = C-X-C Motif Chemokine 12
DAB = Diamino Benzidine
DELFIA = DissociationEnhanced Lanthanide Fluorescent Immunoassay
Delta_CT SH2B3 (ACT SH2B3) = normalized value of SH2B3 gene expression (normalisation vs. housekeeping gene Glycerine aldehyde-3-phosphate-dehydrogenase (GAPDH))
EA = Early Antigen
EC = Endothelial Cells
ECG = Echocardiography
ECLIA = Electrochemiluminescence Sandwich Immunoassay
EDTA = Ethylenediaminetetraacetic Acid
ELISA = Enzyme-Linked Immunosorbent Assay
EMG1 = EMG1 N1-Specific Pseudouridine Methyltransferase
EPC = Endothelial Progenitor Cells, EPC panel, CDs measured in PB
EPO = Erythropoietin
FGF = Fibroblast Growth Factor
GFP = Green Fluorescence Protein
GRB2 = Growth factor receptor-bound protein 2
GMP = Good Manufacturing Practice
HA = Haemagglutinin
HGF = Hepatocyte Growth Factor
HR = Hazard ratio
HIF = Hypoxia-Inducible Factor, transcription factor
ICH GCP = Tripartite Guidelines Guideline for Good Clinical Practice
IGF-1 = Insulin-like Growth Factor 1
IHG = Analysis performed in accordance with *ISHAGE guidelines*
IL = Interleukin
KLF8 = Kruppel Like Factor 8
LMCA = Left Main Coronary Artery
LPCAT2 = Lysophosphatidylcholine Acyltransferase 2
LTB = Lymphotoxin-beta
LVEDV = Left Ventricular End Diastolic Volume
LVEF = Left Ventricular Ejection Fraction
LVESD = Left Ventricular End Systolic Dimension
MACE = Major Adverse Cardiovascular Events
MARK3 = Microtubule Affinity Regulating Kinase 3
MIBI SPECT = Methoxy Iso Butyllsonitrile SPECT
ML = Machine learning
MNC = Mononuclear cells
MRI = Magnetic Resonance Imaging
MS = Mass Spectrometry
6MWT = 6-Minute Walk Test
NBT = 4 Nitro Blue Tetrazolium
NGS = Next Generation Sequencing
NMR = Nuclear Magnetic Resonance
NYHA = New York Heart Association
PB = Peripheral blood
PBMNC = mononuclear cells isolated from peripheral blood
PCI = Percutaneous Coronary Intervention
PDGFRB = Platelet Derived Growth Factor Receptor Beta
PEI = Paul-Ehrlich Institute
PLCG1 = Phospholipase C Gamma 1
PPS = Group of patients for per-protocol set
REX1BD = Required For Excision 1-B Domain
ROC = Receiver Operating Characteristic
RT-RCR = Reverse Transcription Polymerase Chain Reaction
SACM1L = Staphylococcus aureus Cowan 1 phosphatidylinositide phosphatase
SAE = Serious adverse event
SAS = Group of patients for safety set
SDF-1 = Stromal Cell-derived Factor 1
SDS = Sodium Dodecyl Sulphate
SF = Scatter Factor
SH2B3 = SH2 Adapter Protein 3
SCF = Stem Cell FactorSNP = Single Nucleotide Polymorphism
SPA = Scintillation Proximity Assay
STEMI = ST- segment Elevation Infarction
SUM = Support Vector Machines
SUSAR = Suspected Unexpected Serious Adverse Reaction
TNF = Tumor Necrosis Factor
t-SNE = t-distributed stochastic neighbour embedding
VAD = Ventricular Assist Device
VCA = Virus-Capsid-Antigen
VEGF = Vascular Endothelial Growth Factor
VEGF rec = Vascular Endothelial Growth Factor Receptor
ZNF205 = Zinc finger protein 205

## Claims

1. Method for prediction of response to cardiovascular regeneration, wherein the method comprises
(i) determining in a sample of a subject an amount of SH2B3-gene and/or -gene expression product as a biomarker, wherein the SH2B3-gene and/or -gene expression product comprises a mutational variant,
wherein the SH2B3-gene and/or -gene expression variant comprises at least one mutation of SH2B3-gene and/or -gene expression Variant 1 and/or SH2B3-gene and/or -gene expression Variant 2,
wherein the SH2B3-gene and/or -gene expression variant comprises a sequence according to SEQ ID NO: 1 and/or SEQ ID NO: 2,
(ii) comparing the determined amount of SH2B3-gene and/or -gene expression mutational variant product to an amount of a baseline value and/or a reference of SH2B3 non-disease type or individual reference cell without somatic mutation gene and/or -gene expression,
(iii) predicting, based on the results of the comparison whether a response to disease therapy, in particular to tissue repair, preferably to cardiac repair, in the subject is to be expected, not expected or is ambivalent.

2. Method according to claim 1, wherein the SH2B3-gene and/or -gene expression is a knock-out-gene and/or -gene expression variant.

3. Method according to any of the preceding claims, wherein the method further uses analysis of LNK protein expression and/or function of an SH2B3-gene and/or -gene expression variant.

4. Method according to any of the preceding claims, wherein the method further uses one/or more biomarker/s, wherein the further biomarker is selected from the group of Angiogenic factor, Survival factor, Chemokine, circulation endothelial progenitor cells (EPC), circulation endothelial cells (CEC), circulating thrombocytes, circulating mononuclear cells and subpopulations, receptor/ligand expression on MNC subpopulations and/or whole genome sequence RNA.

5. Method according to any of claims, wherein the sensitivity and specificity of prediction accuracy is more than about 90 %, preferably more than about 92 %, more preferably more than about 93 %, most preferably more than about 94 %.

6. Method according to any of the preceding claims, wherein the method is used for preoperative prediction of response to stem cell therapy and/or induction of angiogenesis response and/or tissue repair of a cardiovascular disease including myocardial infarction, stroke and peripheral ischemic vascular disease, heart disease and/or ischemic preconditioning.

7. Method for prediction of response to stem cell therapy according to any of the preceding claims, wherein the sample is taken from a subject suffering from heart disease and/or arteriosclerosis.

8. Method according to any of the preceding claims, wherein the method is used for profiling of angiogenesis response.

9. Method according to any of the preceding claims, wherein the stem cell therapy comprises transplantation of CD133 positive stem cells.

10. Method according to any of the preceding claims, wherein said subject is a human.

11. Method according to any of the preceding claims, wherein said sample is a blood, a serum and/or a plasma sample, and/or a tissue biopsy sample and/or a sample of circulating stem cells such as EPC.

12. A computer device comprising a processor and a memory encoding one or more machine learning (ML) models coupled to the processor, wherein said programs cause the processor to perform a method, said method comprising
(i) comparing the determined amount of biomarker/s according to any of claims 1 to 4 to the amount of a baseline value and/or a reference of SH2B3-gene and/or -gene expression product without mutational variant,
(ii) predicting, based on the results of the comparison, whether a response to cardiovascular regeneration in the subject is to be expected, not expected or is ambivalent.

13. A device adapted for carrying out the method according to any of claims 1 to 11, comprising
(i) an analysing unit for determining in a sample of the subject the amount of each of the biomarkers according to any of claims 1 to 4, and
(ii) a computer device according to claim 12.

## Patentansprüche

1. Methode zur Vorhersage einer Antwort auf kardiovaskulären Regeneration, wobei die Methode umfasst
(i) Bestimmen einer Menge des SH2B3-Gens und/oder des -Genexpressions-Produktes als Biomarker in einer Probe eines Patienten, wobei das SH2B3-Gen und/oder das - Genexpressions-Produkt eine Mutationsvariante umfasst,
wobei die SH2B3-Gen- und/oder die -Genexpressions-Variante zumindest eine Mutation des SH2B3-Gens und/oder der -Genexpressions-Variante 1 und/oder des SH2B3-Gens und/oder der -Genexpressions-Variante 2 umfasst,
wobei das SH2B3-Gen- und/oder die -Genexpressions-Variante eine Sequenz gemäß SEQ ID NO: 1 und/oder SEQ ID NO: 2 umfasst,
(ii) Vergleichen der bestimmten Menge der SH2B3-Gen- und/oder -Genexpressions-Mutations-Variante mit der Menge eines Ausgangswertes und/oder einer Referenz eines SH2B3 nicht-Krankheitstyps oder individuellen Referenzzelle ohne somatisches Mutations-Gens und/oder -Genexpression,
(iii) Vorhersagen, basierend auf den Ergebnissen des Vergleichs, ob eine Antwort auf die Krankheitstherapie, bevorzugt der Reparatur von Gewebe, insbesondere der kardialen Reparatur, in dem Patienten zu erwarten ist, nicht zu erwarten ist oder ambivalent ist.

2. Methode gemäß Anspruch 1, wobei das SH2B3-Gen und/oder die -Genexpression eine knock-out-Gen- und/oder -Genexpressions-Variante ist.

3. Methode gemäß irgendeinem der vorhergehenden Ansprüche, wobei die Methode ferner die Analyse der LNK-Proteinexpression und/oder Funktion einer SH2B3-Gen- und/oder - Genexpressions-Variante umfasst.

4. Methode gemäß irgendeinem der vorhergehenden Ansprüche, wobei die Methode ferner einen oder mehrere Biomarker verwendet, wobei die weiteren Biomarker ausgewählt sind aus der Gruppe von Angiogenem Faktor, Survivalfaktor, Chemokinen, zirkulierenden Endothel-Vorläuferzellen (EPC), zirkulierenden Endothel-Zellen (CEC), zirkulierenden Thrombocyten, zirkulierenden mononukleären Zellen und Subpopulationen und/oder Rezeptor/Ligand Expression auf MNC Subpopulationen und/oder Gesamt-Genom-Sequenz RNA.

5. Methode gemäß irgendeinem der vorhergehenden Ansprüche, wobei die Sensitivität und die Spezifität der Vorhersagegenauigkeit höher als etwa 90 %, bevorzugt höher als etwa 92 %, weiter bevorzugt höher als etwa 93 %, am meisten bevorzugt höher als etwa 94 %, ist.

6. Methode gemäß irgendeinem der vorhergehenden Ansprüche, wobei die Methode für die präoperative Vorhersage der Antwort auf Stammzelltherapie und/oder die Induktion der Angiogenese-Antwort und/oder die Reparatur von Gewebe einer kardiovaskulären Krankheit einschließlich Herzinfarkt, Schlaganfall und periphere ischämische vaskuläre Krankheit, Herzkrankheit und/oder ischämische Präkonditionierung verwendet wird.

7. Methode für die Vorhersage einer Antwort auf Stammzelltherapie gemäß irgendeinem der vorhergehenden Ansprüche, wobei die Probe von einem Patienten entnommen wird, der an einer Herzkrankheit und/oder Arteriosklerose leidet.

8. Methode gemäß irgendeinem der vorhergehenden Ansprüche, wobei die Methode für die Profilerstellung der Angiogenese-Antwort verwendet wird.

9. Methode gemäß irgendeinem der vorhergehenden Ansprüche, wobei die Stammzelltherapie die Transplantation von CD133-positiven Stammzellen umfasst.

10. Methode gemäß irgendeinem der vorhergehenden Ansprüche, wobei der besagte Patient ein Mensch ist.

11. Methode gemäß irgendeinem der vorhergehenden Ansprüche, wobei die besagte Probe eine Blut-, eine Serum- und/oder eine Plasmaprobe und/oder einer Gewebebiopsieprobe und/oder eine Probe von zirkulierenden Stammzellen wie EPC umfasst.

12. Ein Computergerät umfassend einen Prozessor und einen Speicher, der einen oder mehrere maschinelle Lern (ML)-Modelle kodiert, welche an den Prozessor gekoppelt sind, wobei die besagten Programme den Prozessor veranlassen, ein Verfahren durchzuführen, wobei das Verfahren umfasst
(i) Vergleichen der bestimmten Menge von Biomarker/n gemäß irgendeinem der Ansprüche 1 bis 4 mit der Menge eines Ausgangswertes und/oder einer Referenz eines SH2B3-Gens und/oder -Genexpressions-Produktes ohne Mutationsvariante,
(i) Vorhersagen, basierend auf den Ergebnissen des Vergleichs, ob eine Antwort auf die kardiovaskuläre Regeneration in dem Patienten zu erwarten ist, nicht zu erwarten oder ambivalent ist.

13. Ein Computergerät geeignet zur Ausführung des Verfahrens gemäß irgendeiner der Ansprüche 1 bis 11, umfassend
(i) eine Analyseeinheit zur Bestimmung der Menge von jedem der Biomarker gemäß irgendeinem der Ansprüche 1 bis 4 in einer Probe eines Patienten, und
(ii) ein Computergerät gemäß Anspruch 12.

## Revendications

1. Procédé de prédiction de réponse à la régénération cardiovasculaire, dans lequel le procédé comprend les étapes consistant à
(i) déterminer dans un échantillon chez un sujet la quantité du gène SH2B3 et/ou le produit d'expression du gène comme biomarqueur, le gène SH2B3 et/ou le produit d'expression du gène comprenant un variant mutationnel,
le variant du gène SH2B3 et/ou de l'expression du gène comprenant au moins une mutation du variant 1 du gène SH2B3 et/ou de l'expression du gène et/ou du variant 2 du gène SH2B3 et/ou de l'expression du gène,
le variant du gène SH2B3 et/ou de l'expression du gène comprenant une séquence selon SEQ ID n° 1 et/ou SEQ ID n° 2
(ii) comparer la quantité déterminée du variant mutationnel du gène SH2B3 et/ou de l'expression du gène à une valeur de ligne de base et/ou une référence de type non-maladie de SH2B3 ou d'une cellule de référence individuelle sans gène et/ou expression de gène à mutation somatique,
(iii) prédire, sur la base des résultats de la comparaison si une réponse à une thérapie de maladie, en particulier à une réparation de tissu, de préférence à la réparation cardiaque, chez le sujet doit être attendue, non attendue ou est ambivalente.

2. Procédé selon la revendication 1, dans lequel le gène SH2B3 et/ou l'expression du gène est un variant knock-out de gène et/ou d'expression de gène.

3. Procédé selon l'une quelconque des revendications précédentes, dans lequel le procédé utilise en outre l'analyse de l'expression de la protéine LNK et/ou la fonction d'un variant d'un gène SH2B3 ou de l'expression du gène.

4. Procédé selon l'une quelconque des revendications précédentes, dans lequel le procédé utilise en outre un/ou plusieurs biomarqueur/s, l'autre biomarqueur étant choisi dans le groupe constitué par le facteur angiogénique, le facteur de survie, une chimiokine, les cellules progénitrices endothéliales de circulation (EPC), les cellules endothéliales de circulation (CEC), les thrombocytes en circulation, les cellules mononucléaires en circulation et leurs sous-populations, l'expression de récepteur/ligand sur les sous-populations MNC et/ou l'ARN de séquence de génome entier.

5. Procédé selon l'une quelconque des revendications précédentes, dans lequel la sensibilité et la spécificité de précision de prédiction est de plus d'environ 90 %, de préférence de plus d'environ 92 %, davantage de préférence de plus d'environ 93 %, de préférence entre toutes de plus d'environ 94 %.

6. Procédé selon l'une quelconque des revendications précédentes, dans lequel le procédé est utilisé pour la prédiction pré-opératoire de réponse à une thérapie de cellules souches et/ou d'induction de réponse de l'angiogenèse et/ou de la réparation de tissu d'une maladie cardiovasculaire comprenant l'infarctus du myocarde, l'accident vasculaire cérébral et la maladie vasculaire ischémique périphérique, une maladie du coeur et/ou un préconditionnement ischémique.

7. Procédé de prédiction de réponse à une thérapie de cellules souches selon l'une quelconque des revendications précédentes, dans lequel l'échantillon est prélevé d'un sujet souffrant d'une maladie cardiaque ou d'artériosclérose.

8. Procédé selon l'une quelconque des revendications précédentes, dans lequel le procédé est utilisé pour le profilage de la réponse d'angiogenèse.

9. Procédé selon l'une quelconque des revendications précédentes, dans lequel la thérapie de cellules souches comprend la transplantation de cellules souches positives pour CD133.

10. Procédé selon l'une quelconque des revendications précédentes, dans lequel ledit sujet est un être humain.

11. Procédé selon l'une quelconque des revendications précédentes, dans lequel ledit échantillon est un échantillon de sang, de sérum et/ou de plasma et/ou un échantillon de biopsie de tissu et/ou un échantillon de cellules souches en circulation telles que les EPC.

12. Dispositif informatique comprenant un processeur et une mémoire codant pour un ou plusieurs modèles d'apprentissage de machine (ML) couplés au processeur, dans lesquels lesdits programmes entraînent le processeur à effectuer un procédé, ledit procédé comprenant les étapes consistant à
(i) comparer la quantité du biomarqueur/s déterminé(s) selon l'une quelconque des revendications 1 à 4 à la quantité d'une valeur de ligne de base et/ou une référence du gène SH2B3 et/ou du produit de l'expression du gène sans variant mutationnel,
(ii) prédire, sur la base des résultats de la comparaison, si une réponse à la régénération cardiovasculaire, chez le sujet doit être attendue, non attendue ou est ambivalente.

13. Dispositif adapté pour réaliser le procédé selon l'une quelconque des revendications 1 à 11, comprenant
(i) une unité d'analyse pour déterminer dans un échantillon du sujet la quantité de chacun des biomarqueurs selon l'une quelconque des revendications 1 à 4 et
(ii) un dispositif informatique selon la revendication 12.
